# FASCICULE DE BREVET EUROPEEN

(11) **EP 1 174 120 B1**
(45) Date de publication et mention de la délivrance du brevet: **16.11.2005**
(21) Numéro de dépôt: 01401582.0
(22) Date de dépôt: 15.06.2001
(51) Int. Cl.: A61K 7/48, A61K 35/78

(54) **Utilisation d'un extrait d'une Iridacée dans une composition destinée à stimuler les défenses immunitaires**
Verwendung eines Iridaceae-Extrakts in einer Zusammensetzung für die Stimulierung der Immunabwehr
Use of an extract of an Iridaceae in an immune defenses stimulating composition

(30) Priorité: 18.07.2000 FR 0009404
(43) Date de publication de la demande: 23.01.2002
(73) Titulaire: L'OREAL, 75008 Paris (FR)
(72) Inventeur: Breton, Lionel, 78000 Versailles (FR); Gueniche, Audrey, 78140 Velizy (FR)
(74) Mandataire: Allab, Myriam

(56) Documents cités:
- EP-A- 0 765 668
- FR-A- 2 746 645
- US-A- 5 382 430
- DATABASE WPI Week 200017 Derwent Publications Ltd., London, GB; AN 2000-194093 XP002163376 & RU 2 123 349 C (ST PETERSBURG CHEM PHARM ACAD), 20 décembre 1998 (1998-12-20)

## Description

L'invention concerne l'utilisation d'au moins un extrait de cellules dédifférenciées d'au moins une Iridacée du genre Iris pour préparer une composition destinée à stimuler les défenses immunitaires de la peau affaiblies par les rayonnements ultraviolets, induisant à long terme un vieillissement photo-induit.

Le système immunitaire comprend un ensemble de cellules spécialisées soumises à de multiples mécanismes de contrôle assurant leur renouvellement, leur activation et leur différenciation, indispensables à un niveau normal d'immunocompétence. Le rôle du système immunitaire est de reconnaître le soi du non soi pour éliminer les agents pathogènes et les tumeurs spontanées. Toute déplétion cellulaire, toute dysrégulation immunitaire ou tout déficit fonctionnel est susceptible de favoriser la survenue de manifestations pathologiques caractérisées par la perturbation des mécanismes de reconnaissance du soi vis-à-vis du non soi, et une plus grande sensibilité vis-à-vis des agressions microbiennes et des processus néoplasiques.

La peau constitue l'organe le plus important de l'organisme et est reconnue comme un élément actif du système de défense immunitaire. Trois types de cellules épidermiques participent à ce système : les kératinocytes, les mélanocytes et les cellules de Langerhans. Ces cellules que l'on ne retrouve qu'au niveau de l'épiderme, jouent un rôle primordial dans la réponse immunitaire cutané.

La peau saine est capable de se défendre des agressions extérieures grâce aux moyens mis à sa disposition. Néanmoins, elle est soumise à l'agression permanente de l'environnement (infections et pollution notamment), de produits chimiques, de radiations. En particulier, les cellules de Langerhans sont la cible privilégiée des rayonnements ultraviolets.

Ces agressions se traduisent par un effet suppresseur sur les défenses immunitaires entraînant une baisse des résistances aux agents pathogènes et notamment, une augmentation de l'incidence de certains cancers.

Pour aider la peau à remplir sa fonction immune, des produits de régulation du système immunitaire cutanée sont d'un grand intérêt.

De ce qui précède, on sait maintenant que le système immunitaire et plus particulièrement celui de la peau s'affaiblit au cours du vieillissement chronobiologique ou du vieillissement photo-induit.
Un effet immunorégulateur appliqué sur ce système immunitaire peut donc rétablir les fonctions immunitaires et plus particulièrement celles de l'épiderme ce qui concourre à restaurer les défenses naturelles de la peau et à prévenir ainsi de certaines affections pathogènes et de l'apparition de cancers.

Un des buts de l'invention est donc de proposer un nouvel immunorégulateur stimulant, plus particulièrement un régulateur stimulant du système immunitaire de la peau compatible avec cette dernière.

Le brevet US 5 382 430 décrit l'utilisation de glycolipides de poids moléculaire égal à 8000 ± 1000, extraits de plantes, telles que les Monocotylédones, Dicotylédones et Gymnospermes. Ces glycolipides sont positifs au « limulus test » et jouent un rôle dans la stimulation du système immunitaire par l'activation des macrophages.

La demanderesse a trouvé de manière inattendue que l'application d'une quantité efficace d'au moins un extrait d'au moins une Iridacée permet lors des agressions de ce système de stimuler les défenses immunitaires de la peau.
L'application de l'extrait est particulièrement efficace dans la stimulation des défenses immunitaires de la peau lorsque celles-ci sont affaiblies par des rayonnements ultraviolets, induisant à long terme un vieillissement photo-induit.

L'invention a donc pour objet l'utilisation d'au moins un extrait de cellules dédifférenciées d'au moins une Iridacée du genre Iris pour préparer une composition destinée à stimuler les défenses immunitaires de la peau affaiblies par les rayonnements ultraviolets, induisant à long terme un vieillissement photo-induit.

La famille des Iridacées compte environ 750 espèces.
Les plantes de la famille des Iridacées sont surtout utilisées pour leurs propriétés aromatiques et ornementales.

Selon l'invention, on utilise du matériel végétal issu du genre Iris et préférentiellement du matériel végétal issu des espèces *Iris pallida, Iris germanica* et *Iris florentina.*

L'extrait d'au moins une Iridacée peut être tout extrait préparé à partir de tout matériel végétal issu de la famille des Iridacées.
Le matériel végétal peut provenir de plantes entières cultivées *in vivo* ou issues de culture *in vitro.*
La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Par culture *in vitro,* on entend l'ensemble des techniques connues de l'homme du métier qui permet de manière artificielle l'obtention d'un végétal ou d'une partie d'un végétal.

De préférence selon l'invention, on utilise un extrait obtenu à partir de matériel végétal cultivé *in vitro.*

Le matériel végétal utilisable selon l'invention est issu des cellules végétales dédifférenciées.

Par cellules végétales dédifférenciées, on entend toute cellule végétale ne présentant aucun des caractères d'une spécialisation particulière et capable de vivre par elle-même et non en dépendance avec d'autres cellules. Ces cellules végétales dédifférenciées sont éventuellement aptes, sous l'effet d'une induction, à toute différenciation conforme à leur génome.

Les cellules végétales dédifférenciées peuvent être obtenues à partir de matériel végétal issu de plante entière ou de partie de plante comme les feuilles, les tiges, les fleurs, les pétales, les racines, les fruits, les graines, les anthères, que la plante ou la partie de plante ait été obtenue par culture *in vivo* ou par culture *in* *vitro.*

Préférentiellement, les cellules végétales dédifférenciées sont obtenues à partir de feuilles ou des rhizomes.

Préférentiellement selon l'invention, on utilise des cellules végétales dédifférenciées issues de culture *in vitro*.

D'une manière très préférentielle, on utilise selon l'invention des cellules végétales dédifférenciées issues de culture *in vitro* obtenues à partir de feuilles ou des rhizomes d'un végétal des espèces *Iris pallida, Iris germanica* et *Iris florentina*.

Les cellules végétales dédifférenciées utilisables selon l'invention peuvent être obtenues par toute méthode connue de l'art antérieur. A cet égard on peut citer les méthodes décrites par E.F. George et P.D. Sherrington dans Plant Propagation by tissue culture, handbook and directory of commercial laboratories (Exegetics Ltd 1984).
Les milieux de culture utilisables selon l'invention sont ceux généralement connus de l'homme du métier. On peut citer à titre d'exemples les milieux de Gamborg, Murashige et Skoog, Heller, White etc.... On trouvera dans "Plant Culture Media : formulations and uses" de E.F. George, DJM Puttock et H.J George (Exegetics Ltd 1987, tome 1 & 2) des descriptions complètes de ces milieux.

Préférentiellement selon l'invention on prépare les cellules végétales dédifférenciées cultivées sur milieu de Murashige et Skoog.

Toute méthode d'extraction connue de l'homme du métier peut être utilisée pour préparer l'extrait d'Iridacée utilisable selon l'invention.
On peut, en particulier, citer les extraits alcooliques, notamment éthanoliques ou encore hydroalcooliques.

On peut également utiliser un extrait d'Iridacée préparé par la méthode décrite dans la demande de brevet français n° 95-02379 déposée par la demanderesse. Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites. Le produit de cette extraction aqueuse peut également être lyophilisé pour obtenir un extrait sec (en moyenne selon la nature de la source initiale, on retrouve entre 5 et 70 g de matière sèche par litre d'extrait aqueux).

Un exemple de préparation d'extrait d'Iridacée utilisable selon l'invention est donné par ailleurs dans les exemples.

La quantité d'extrait utilisable selon l'invention est bien entendu fonction de l'effet recherché et peut donc varier dans une large mesure.

Pour donner un ordre de grandeur, on peut utiliser un extrait sec tel que défini précédemment en une quantité représentant de 0,0001 % à 20 % du poids total de la composition et préférentiellement en une quantité représentant de 0,001 % à 10 % du poids total de la composition.

Les compositions utilisables selon l'invention sont des compositions à usage cosmétique ou dermatologique.

Les compositions utilisables selon l'invention peuvent se présenter sous toutes les formes galéniques normalement utilisées pour une application topique ou orale.

Selon l'invention, on préfère des compositions à application topique.

Pour une application topique, les compositions de l'invention comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de traitement ou de soin cosmétique ou pharmaceutique pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Les quantités des différents constituants des compositions selon l'invention sont celles classiquement utilisées dans les domaines considérés et sont appropriées à leur forme galénique.

Quand la composition est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou pharmaceutique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids, et de préférence de 0,5 à 30 % en poids par rapport au poids total de la composition.

De façon connue, les compositions peuvent contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou pharmaceutique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions, on peut notamment citer les actifs ayant un effet sur le traitement des rides ou des ridules, et en particulier les actifs kératolytiques. Par kératolytique, on entend un actif ayant des propriétés desquamantes, exfoliantes ou gommantes ou un actif capable de diminuer la cohésion de la couche cornée.

Parmi les actifs ayant un effet sur le traitement des rides ou des ridules que peuvent contenir les compositions, on peut en particulier citer les hydroxyacides et les rétinoïdes.

Les hydroxyacides peuvent être par exemple des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Parmi les actifs que peuvent contenir les compositions, on peut notamment citer les actifs qui vont participer à la protection du système immunitaire.

Parmi les actifs capables de participer à la protection du système immunitaire, on peut notamment citer les actifs antiradicaux libres, comme par exemple les caroténoïdes provitaminiques A, comme le Béta-Carotène, les caroténoïdes non provitaminiques A, comme le Lycopène, la vitamine E et ses dérivés, la vitamine C et ses dérivés, les filtres solaires minéraux et organiques.

Parmi les actifs que peuvent également contenir les compositions on peut notamment citer les filtres solaires, les actifs qui protègent les protéines de la matrice extracellulaire des dégâts UV induits, comme les inhibiteurs de protéases UV induits, notamment les inhibiteurs de métalloprotéinases 1, cette inhibition enzymatique peut s'exprimer sur l'activité enzymatique mais également sur la synthèse de l'enzyme proprement dite. On citera notamment les antiAP1 tels que décrits dans le brevet US 5837224 de J. Voorhees et al.

Ces actifs peuvent être utilisés en particulier à des concentrations allant de 0,0001 % à 5% en poids par rapport au poids total de la composition.
Quand les compositions sont destinées à une application par voie orale, elles peuvent se présenter sous les formes galéniques habituelles dans ce domaine, telles que les comprimés, les gélules, les produits buvables, notamment constitués extemporanément, les granulés, les poudres, dans les excipients habituels pour une telle application.

Les exemples et compositions suivants illustrent l'invention. Dans les compositions les proportions indiquées sont des pourcentages en poids, sauf mention contraire.

### Exemple 1 : Préparation d'un extrait d'Iris pallida :

Des cellules dédifférenciées d'*Iris pallida* cultivées *in vitro* en conditions axéniques sont récupérées après culture en Erlenmeyer ou en fermenteur par filtration sur tamis de 50µm. A 55 g de matière fraîche ainsi obtenue, on ajoute 27,5 ml d'eau déminéralisée. L'ensemble est broyé au Turax à 24000 T/min durant 1 minute à 4°C (bain de glace). Le broyât est centrifugé 15 à 10000 G à 4°C. Le surnageant est filtré à 0,22 µm (filtration stérilisante).
L'extrait ainsi préparé est conservé à 4°C. Il renferme environ 15 g de matière sèche par litre et il peut être lyophilisé pour obtenir un extrait sec.
Si le matériel végétal est de la plante entière, on ramène la matière fraîche à traiter en fonction du poids sec pour se mettre dans les mêmes conditions d'extraction que pour l'*in vitro.* Les différentes parties de la plante sont prélevées en fonction du poids relatif de chaque partie de celle-ci. Le traitement à froid permet de geler les activités enzymatiques, la filtration stérilisante évite la dégradation des actifs par les micro-organismes de l'environnement. Enfin, le véhicule eau est compatible avec les récepteurs ex vivo et facilite les formulations cosmétiques ou pharmaceutiques. L'extrait peut également être utilisé sous forme d'extrait sec.

### Exemple 2 : Activité d'un extrait d'Iris pallida (extrait sec obtenu par lyophilisation de l'extrait aqueux décrit dans l'exemple 1) sur la protection du système immunitaire de la peau déprimé après irradiation UV.

L'extrait d'*Iris pallida* a été appliqué chez la souris hairless Skh/hr1 après une irradiation par un simulateur solaire (Xenon 1000 W (Oriel) équipé de filtres (Oriel 81017 + 81019)) à une dose connue comme provoquant une inhibition de l'hypersensibilité de contact (HSC). L'irradiation, mesurée avec un radiomètre ARCC 1600/OSRAM, était de 2,0 mW/cm² en UVB et de 9,4 mW/cm² en UVA. Des biopsies cutanées sont ensuite réalisées 14 jours après l'irradiation.

Les produits appliqués sont formulés dans un support simple :
- Véhicule témoin : gel AMPS-604518, vendu par la société CLARIANT (FRANCE) sous la dénomination HOSTACERIN AMPS® .
- *Iris pallida* à 5 % (extrait sec) dans un gel AMPS-604518.

Les résultats obtenus montrent que dans le système utilisé (souche murine hairless Skh/hr1, simulateur solaire (irradiation aiguë à une dose de 2,5 MED), HSC systémique au DNFB, l'application d'un extrait sec d'*Iris pallida* formulé à 5 % dans un gel AMPS atténue significativement les effet immunosuppresseurs engendrés par les UVs mesurés par une réaction d'HSC au dinitrofluorobenzène (DNCB).

En effet, les produits sans posséder d'activité stimulatrice sur la réaction d'HSC, réduisent la photoimmunosuppression.

Parallèlement à cet examen clinique, une mesure histologique/ biochimique (nombre, morphologie) des cellules de Langerhans a été réalisée.

Chez les témoins non protégés par *Iris pallida* environ 50 % du nombre de cellules de Langerhans disparaissent après irradiation UV avec pour celles qui restent, des altérations importantes au niveau de la surface cellulaire et de la morphologie.

Le traitement par l'extrait d'*Iris pallida* protège la peau de l'altération des cellules de Langerhans induite par l'irradiation puisque le nombre et l'aspect morphologique de ces cellules est identique au contrôle (cf. Documents photographiques joints et tableau ci-dessous).

Quantification des cellules de Langerhans au cours du traitement donné en nombre de celles de Langerhans par mm² :

| Témoins non irradiés | Témoins irradiés | Traités non irradiés | Traités irradiés |
|---|---|---|---|
| 1406 ± 82 | 720 ± 36 | 1334 ± 97 | 1515 ± 112 |

Chez les témoins irradiés, environ 50 % des cellules de Langerhans disparaissent. Cette disparition est inhibée par l'application d'un gel contenant 5 % d'un extrait sec d'*Iris pallida.* Par ailleurs, l'application du gel d'*Iris pallida* à 5 % n'a d'influence sur le système immunitaire cutané que lorsque ce dernier est altéré par les UVs.

### Exemple 3 : Exemples de formulations illustrant l'invention. Ces compositions ont été obtenues par simple mélange des différents composants.

### Composition 1 : Lait pour le visage

- Huile de vaseline 7,0 %
- Extrait de l'exemple 1 (extrait Lyophilisé) 5,0 %
- Monostéarate de glycéryle, stéarate de polyéthylène glycol (100 OE) 3,0 %
- Polymère carboxyvinylique 0,4 %
- Alcool stéarylique 0,7 %
- Protéines de soja 3,0 %
- NaOH 0,4 %
- Conservateur qs
- Eau qsp 100,0 %

Cette composition se présente sous forme d'un lait pour le visage, ayant de bonnes propriétés cosmétiques, doux et confortable à utiliser.
Le pH de la composition est d'environ 5,5.

### Composition 2 : Lotion

- Extrait de l'exemple 1 (extrait Lyophilisé) 2,0 %
- Palmitate d'éthyl-2 hexyle 10,0 %
- Cyclopentadiméthylsiloxane 20,0 %
- Butylène glycol 5,0 %
- Conservateur qs
- Eau qsp 100,0 %

Cette lotion, qui ne contient pas de tensioactif, favorise la desquamation de la peau.

### Composition 3 : Lait

- Palmitate d'octyle 35,0 %
- Glycérine 2,0 %
- Extrait de l'exemple 1 (extrait Lyophilisé) 3,0 %
- Polymère réticulé acrylates C10-C30/alkylacrylates 0,1 %
- Triéthanolamine 0,1 %
- Acides aminés de blé 1,0 %
- Conservateur qs
- Eau qsp 100,0 %

Le lait obtenu, qui ne contient pas de tensioactif, possède de bonnes propriétés cosmétiques. ,

### Composition 4 : Gel pour le visage

- Glycérine 10,0 %
- Extrait de l'exemple 1 (extrait Lyophilisé) 5,0 %
- Cocoamphodiacétate de disodium 1,0 %
- Conservateur qs
- Eau qsp 100,0 %

Le gel obtenu possède de bonnes propriétés cosmétiques.

### Composition 5 : Gel nettoyant à l'eau

- Butylène glycol 7,0 %
- Sarcosinate de lauroyl sodium 4,0 %
- Extrait de l'exemple 1 (extrait Lyophilisé) 1,5 %
- Triéthanolamine 0,8 %
- Carbomer 0,5 %
- Conservateur qs
- Eau qsp 100,0 %

Le gel obtenu possède de bonnes propriétés cosmétiques.

## Revendications

1. Utilisation d'au moins un extrait de cellules dédifférenciées d'au moins une Iridacée du genre Iris pour préparer une composition destinée à stimuler les défenses immunitaires de la peau affaiblies par les rayonnements ultraviolets, induisant à long terme un vieillissement photo-induit.

2. Utilisation selon la revendication précédente, **caractérisée en ce que** l'extrait est obtenu par culture *in vitro.*

3. Utilisation selon l'une quelconque des revendications précédentes, **caractérisée en ce que** ledit extrait est un extrait d'un végétal d'une espèce choisie parmi *Iris pallida, Iris florentina* ou *Iris germanica*.

4. Utilisation selon l'une quelconque des revendications précédentes, **caractérisée en ce que** dans la composition ledit extrait d'Iridacée est utilisé sous sa forme lyophilisée en une quantité représentant de 0,0001 % à 20 % du poids total de la composition.

5. Utilisation selon la revendication précédente, **caractérisée en ce que** dans la composition ledit extrait d'Iridacée est utilisé en une quantité représentant de 0,001 % à 10 % du poids total de la composition.

6. Utilisation selon l'une quelconque des revendications 1 à 5, **caractérisée en ce que** la composition peut être appliquée par voie topique ou orale.

## Patentansprüche

1. Verwendung mindestens eines Extrakts von undifferenzierten Zellen mindestens einer Iridacea der Gattung Iris zur Herstellung einer Zusammensetzung, die dazu vorgesehen ist, die Immunabwehr der Haut zu stimulieren, die durch UV-Strahlung geschwächt wurde, durch die langfristig die lichtinduzierte Alterung ausgelöst wird.

2. Verwendung nach dem vorhergehenden Anspruch, **dadurch gekennzeichnet, dass** der Extrakt durch *in vitro* Kultur erhalten wird.

3. Verwendung nach einem der vorhergehenden Ansprüche, **dadurch gekennzeichnet, dass** der Extrakt ein Extrakt einer Pflanzenart ist, die unter *Iris pallida, Iris florentina* oder *Iris germanica* ausgewählt ist.

4. Verwendung nach einem der vorhergehenden Ansprüche, **dadurch gekennzeichnet, dass** der Iridacea-Extrakt in der Zusammensetzung in lyophilisierter Form in einer Menge von 0,0001 bis 20 % des Gesamtgewichts der Zusammensetzung verwendet wird.

5. Verwendung nach dem vorhergehenden Anspruch, **dadurch gekennzeichnet, dass** der Iridacea-Extrakt in der Zusammensetzung in einer Menge von 0,001 bis 10 % des Gesamtgewichts der Zusammensetzung verwendet wird.

6. Verwendung nach einem der Ansprüche 1 bis 5, **dadurch gekennzeichnet, dass** die Zusammensetzung auf topischem oder oralem Weg angewandt wird.

## Claims

1. Use of at least one extract of dedifferentiated cells of at least one Iridacea of the Iris genus, for preparing a composition intended to stimulate the immune defences of the skin that have been weakened by ultraviolet radiation, inducing, in the long term, photo-induced ageing.

2. Use according to the preceding claim, **characterized in that** the extract is obtained by culturing *in vitro.*

3. Use according to either of the preceding claims, **characterized in that** said extract is an extract of a plant of a species chosen from *Iris pallida, Iris florentina* or *Iris germanica*.

4. Use according to any one of the preceding claims, **characterized in that**, in the composition, said extract of Iridacea is used in its lyophilized form in an amount representing from 0.0001% to 20% of the total weight of the composition.

5. Use according to the preceding claim, **characterized in that**, in the composition, said extract of Iridacea is used in an amount representing from 0.001% to 10% of the total weight of the composition.

6. Use according to any one of Claims 1 to 5, **characterized in that** the composition can be applied topically or orally.
